# EUROPEAN PATENT SPECIFICATION

(11) **EP 4 233 980 B1**
(45) Date of publication and mention of the grant of the patent: **25.03.2026**
(21) Application number: 22216209.1
(22) Date of filing: 22.12.2022
(51) Int. Cl.: A61M 39/00, A61M 39/06, A61M 25/00, A61M 25/01

(54) **INTEGRATED HEMOSTASIS BYPASS VALVE**
INTEGRIERTES HÄMOSTATISCHES BYPASS-VENTIL
VALVE DE DÉRIVATION HÉMOSTATIQUE INTÉGRÉE

(30) Priority: 28.02.2022 US 202263314516 P
(43) Date of publication of application: 30.08.2023
(60) Divisional application: 25226658.0
(73) Proprietor: St. Jude Medical, Cardiology Division, Inc., St. Paul MN 55117 (US)
(72) Inventor: Eidenschink, Tracee, Wayzata, 2232 (US); Glimsdale, Mathias C., St. Michael, 55376 (US); Perszyk, Brian Joseph, Shoreview, 55126 (US); Osterbauer, Philip, Wyoming, 55092 (US)
(74) Representative: LKGLOBAL Partnerschaftsgesellschaft mbB

(56) References cited:
- WO-A1-99/34849
- US-A- 5 911 710
- US-A1- 2009 270 813
- US-A1- 2020 390 549
- US-B1- 6 276 661
- US-B2- 9 320 503

## Description

### Background

Catheters are frequently used to assist in the delivery of medical devices into a patient non-invasively. For example, several types of collapsible and expandable medical devices may be delivered to, and implanted within, the heart of a patient using a catheter that is advanced through the vasculature and into the patient's heart without needing to make any incisions in the patient's chest or heart, and without needing to put the patient on cardiopulmonary bypass.

Left atrial appendage ("LAA") occluder devices are one example of collapsible and expandable medical devices that may be delivered to a patient's heart via a catheter that traverses the patient's vasculature. In some examples, a catheter may be advanced through the patient's femoral vein, into the right atrium through the inferior vena cava, across the atrial septum and into the left atrium, with a distal end of the catheter positioned within or adjacent to the LAA. The LAA occluder device may be within the catheter during the advancement of the catheter, or otherwise may be advanced through the catheter after the catheter is already in the desired position. The LAA occluder may be in a collapsed state with a relatively small profile while inside the catheter, and may self-expand into the LAA upon deployment from the distal end of the catheter. One such LAA occluder is the Amplatzer^{™} Amulet^{™} Occluder offered by Abbott Labs. One example of a LAA occluder device is described in U.S. Patent No. 10,201,337.

US 2020/0390549 A1 describes an introducer device and methods for introducing a medical device into a patient's vasculature. The introducer device may comprise a housing, a hemostatic seal mounted within the housing, and a tube extending into the housing and movable longitudinally relative to the hemostatic seal to a position such that the distal end of the tube extends through the hemostatic seal. US 5 911 710 A describes a medical insertion device that reduces the drag force exerted on an elongated member such as a catheter that is inserted through the device. Further, WO 99/34849 A1 is directed to a catheter sheath introducer for reducing friction, US 9 320 503 B2 is directed to devices, systems and methods for guiding an operative tool into an interior body region, US 2009/270813 A1 is directed to a wicking fluid management in a surgical access device, and US 6 276 661 B1 is directed to a pressure actuated introducer valve.

As explained in greater detail below, although this disclosure generally focuses on a hemostasis bypass valve in the context of a steerable sheath for delivering a LAA occlude, the disclosure is not so limited, and may apply to various other types of sheaths (including non-steerable sheaths) and various other types of medical devices to be delivered (including other occlude-type devices, such as PFO closure devices, and other devices that are not occluders).

### Brief Summary

A delivery device according to the invention is defined by the independent claim 1. Embodiments thereof are defined by the dependent claims.

According to one aspect of the disclosure, a delivery device may include a handle, a catheter sheath extending distally from the handle, and a hemostasis valve positioned within the handle. The hemostasis valve may be located proximal to the catheter sheath and distal to a proximal end of the handle. The delivery device may also include a hemostasis bypass assembly coupled to the handle. The hemostasis bypass assembly may include a bypass tube coupled to an actuator. The actuator may be configured to be transitioned between a first condition in which a distal end of the bypass tube is positioned proximal to the hemostasis valve and the hemostasis valve is closed, and a second condition in which the distal end of the bypass tube traverses the hemostasis valve and the hemostasis valve is opened. A wiper seal may be coupled to the hemostasis bypass assembly proximal to the hemostasis valve.

According to another aspect of the disclosure, a delivery device includes a handle, a catheter sheath extending distally from the handle, and a hemostasis valve positioned within the handle. The hemostasis valve may be located proximal to the catheter sheath and distal to a proximal end of the handle. A hemostasis bypass assembly may be coupled to the handle. The hemostasis bypass assembly may include a bypass tube coupled to an actuator. The actuator may be configured to be transitioned between a first condition in which a distal end of the bypass tube is positioned proximal to the hemostasis valve and the hemostasis valve is closed, and a second condition in which the distal end of the bypass tube traverses the hemostasis valve and the hemostasis valve is opened. The actuator may be a rotatable knob.

According to still another aspect of the disclosure, a delivery device includes a handle, a catheter sheath extending distally from the handle, and a hemostasis valve positioned within the handle. The hemostasis valve may be located proximal to the catheter sheath and distal to a proximal end of the handle. A hemostasis bypass assembly may be coupled to the handle. The hemostasis bypass assembly may include a bypass tube coupled to an actuator. The actuator may be configured to be transitioned between a first condition in which a distal end of the bypass tube is positioned proximal to the hemostasis valve and the hemostasis valve is closed, and a second condition in which the distal end of the bypass tube traverses the hemostasis valve and the hemostasis valve is opened. The actuator may be axially translatable between the first condition and the second condition.

### Brief Description of the Drawings

Fig. 1 is a schematic view of a steerable sheath according to one aspect of the disclosure.
Fig. 2 is a schematic view of a dilator for use with the steerable sheath of Fig. 1.
Fig. 3A is a perspective view of a hemostasis valve assembly of the steerable sheath of Fig. 1.
Fig. 3B is an exploded view of the hemostasis valve assembly of Fig. 3A.
Fig. 3C is a side view of the hemostasis valve assembly of Fig. 3A.
Fig. 3D is a cross-section of the hemostasis valve assembly of Fig. 3A taken along the section line 3D-3D of Fig. 3C.
Fig. 3E is an enlarged isolated view of portion 3E of Fig. 3D.
Figs. 3F is a top perspective view of a hemostasis valve of the hemostasis valve assembly of 3A.
Figs. 3G-H are side and top views, respectively, of the hemostasis valve of Fig. 3F illustrating slits forming the valve functionality.
Fig. 4A is perspective view of the hemostasis valve assembly of Fig. 3A assembled to a hemostasis valve knob.
Fig. 4B is an exploded view of the hemostasis valve assembly and the hemostasis valve knob of Fig. 4A.
Fig. 4C is a side view of the hemostasis valve assembly and knob of Fig. 4A.
Fig. 4D is a cross-section of the hemostasis valve assembly and knob of Fig. 4A taken along the section line 4D-4D of Fig. 4C.
Fig. 4E is a cross-section of the hemostasis valve assembly and knob of Fig. 4A taken along the section line 4E-4E of Fig. 4C, with certain components omitted from the view.
Fig. 4F is a cross-section of the hemostasis valve assembly and knob of Fig. 4A taken along the section line 4D-4D of Fic. 4C after activation of the valve bypass feature.
Fig. 5A is a perspective view of an occluder in an expanded or deployed condition.
Fig. 5B is a schematic view of the occluder of Fig. 5A in a collapsed or delivery condition.
Fig. 5C is a side view of the occluder of Fig. 5A coupled to a delivery cable.
Fig. 5D is a highly schematic view of the occluder of Fig. 5A being passed through a loading tube coupled to the hemostasis valve knob and hemostasis valve assembly of Fig. 4F.
Fig. 6A is a side view of a main body of a hemostasis valve knob according to another aspect of the disclosure.
Fig. 6B is a side view of the main body of Fig. 6A assembled to a portion of the hemostasis valve assembly of Fig. 3A.
Fig. 6C is a cut-away view of an alternate version of the main body of the hemostasis valve knob of Fig. 6A, according to the invention.
Fig. 7A is a perspective view of a hemostasis valve knob assembled to a hemostasis valve assembly in a non-bypassed condition, according to another aspect of the disclosure.
Fig. 7B is a perspective view of the hemostasis valve knob of Fig. 7A assembled to the hemostasis valve assembly of Fig. 7A in a bypassed condition.
Fig. 7C is a cross-section of the hemostasis valve assembly of Figs. 7A-B.
Fig. 7D is a perspective view of the hemostasis valve knob of Figs. 7A-B.
Fig. 7E is a perspective view of a plug of the hemostasis valve assembly of Fig. 7C.
Fig. 7F is a cross-section of the hemostasis valve knob of Fig. 7D assembled to the hemostasis valve assembly of Fig. 7C in the bypassed condition.
Fig. 8A is a perspective view of a wiper seal.
Fig. 8B is a cross-section of the wiper seal of Fig. 8A assembled to a portion of the hemostasis valve assembly of Fig. 3D.
Fig. 9A is front view of a flat wiper seal.
Fig. 9B is a cross-section of the flat wiper seal of Fig. 8A assembled to the hemostasis valve assembly of Fig. 3D.
Fig. 9C illustrates the relative positioning of the wiper seal of Fig. 8A being used in conjunction with the wiper seal of Fig. 9A, with other components of the hemostasis valve assembly omitted for clarity.
Fig. 10 is a cross-section of the hemostasis valve assembly, according to another embodiment of the disclosure, assembled to the hemostasis valve knob.
Fig. 11 is an enlarged isolated view of a portion of an alternate version of the hemostasis valve assembly, similar to the view of Fig. 3E.
Figs. 12A-B are cross-sections of the hemostasis valve assembly shown in Fig. 3D with alternate flush port designs.

### Detailed Description

As used herein, the term proximal refers to a position relatively close to a user of a medical device, while the term distal refers to a position relatively far from the user of the medical device, when the medical device is being used in an intended manner. In other words, the leading end of a medical device is positioned distal to the trailing end of the medical device.

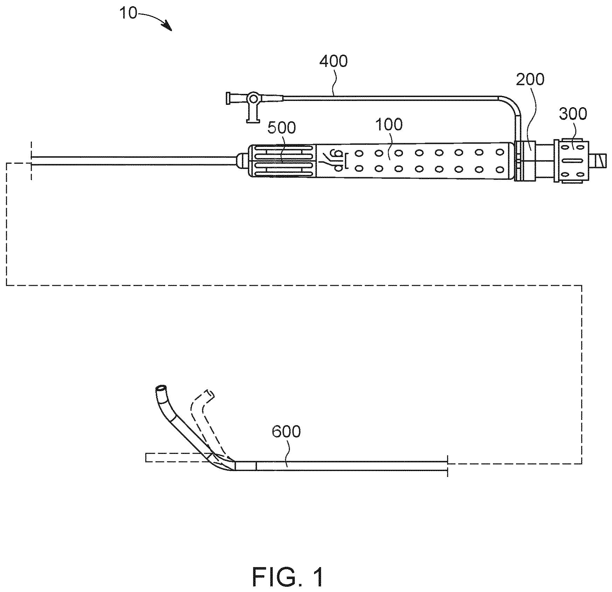

Fig. 1 illustrates a delivery device 10, which in the illustrated embodiment is a steerable sheath, although it should be understood that the inventive concepts disclosed herein may be used in conjunction with other catheter or sheath devices, whether or not steerable. Generally, delivery device 10 includes a handle 100, a hemostasis valve assembly 200 at a proximal end of the handle 100, a hemostasis valve knob (or actuator) 300, a flushing tube 400, a deflection knob 500 at a distal end of the handle 100, and a deflectable sheath 600 extending from a distal end of the deflection knob 500 to a terminal distal end of the delivery device 10. This disclosure focuses on the hemostasis valve assembly 200 and the hemostasis valve knob 300, and the remainder of the delivery device 10 is generally described for the purpose of providing a contextual example of the use of the hemostasis valve assembly 200 and hemostasis valve knob 300. Thus, it should be understood that the hemostasis valve assembly 200 and hemostasis valve knob 300 described herein (including the optional variations described therewith) may be applicable to any suitable delivery device in which a hemostasis valve is desired. Delivery device 10 is particularly suited for delivery of a collapsible and expandable LAA occluder, but it should be understood that the delivery device 10 may be suited to delivery of other medical devices.

The handle 100 may be a generally cylindrical or otherwise shaped member that the user of the delivery device 10 may grip during use. The handle 100 may be at least partially hollow and house various components therein, and may have one or more internal lumens so that medical devices may be passed through the delivery device 10 from the proximal end to and beyond the terminal distal end of the delivery device 10. The handle 100 may be rotatably coupled to the deflection knob 500, with the deflection knob 500 being rotatable about the central longitudinal axis of the handle 100. The deflection knob 500 may be operably coupled to two pull wires that traverse the deflectable sheath 600 and which are fixed to anchors (or pull rings or similar structures) near the distal tip of the sheath 600. Rotation of the deflection knob 500 in a first rotational direction may deflect the distal tip of the sheath 600 in a first deflection direction, while rotation of the deflection knob 500 in a second opposite rotational direction may deflect the distal tip of the sheath 600 in a second deflection direction opposite the first deflection direction. In one exemplary embodiment, the distal tip of the sheath may have a neutral angled position of about 45 degrees relative to the central longitudinal axis of the delivery device 10, with a maximum deflection (upon rotation of the deflection knob 500 in the first, e.g. clockwise, rotational direction) of about 120 degrees (shown in phantom lines in Fig. 1) relative to the central longitudinal axis of the delivery device 10, and a minimum deflection (upon rotation of the deflection knob 500 in the second, e.g. counterclockwise, rotational direction) of about 0 degrees (shown in phantom lines in Fig. 1) relative to the central longitudinal axis of the delivery device 10. In one example, the proximal end of each pull wire may be coupled to an axially slideable component within handle 100, where rotation of the deflection knob 500 causes the two axially slideable components to slide axially in opposite directions. Suitable pull wire mechanisms are described in greater detail in U.S. Patent No. 7,691,095. The deflection mechanisms and ranges described above are merely exemplary, and as noted above, steering or deflection control may in some embodiments be entirely omitted from delivery device 10.

The catheter 600 may define a lumen therethrough configured to allow other devices to pass through the lumen. The catheter 600 may be formed from any suitable materials and in any suitable configuration. In one example, the catheter 600 includes an innermost liner layer, a torque transfer layer surrounding at least portions of the inner layer, and an outer sheath formed over the torque transfer layer. The wall of the catheter 600 may define lumens as well, for example two lumens spaced about 180 degrees apart, to accommodate the pull wires therethrough. Examples of suitable methods and materials for use in forming the catheter 600 are described in greater detail in U.S. Patent No. 7,914,515. However, it should be understood that the hemostasis valve assembly 200 and hemostasis valve knob 300 may be used with any suitable catheter configuration.

Flushing tube 400 may be a tube with a valve *(e.g.* luer lock) or connector at a proximal end thereof, with the distal end of the flushing tube 400 being in fluid communication with hemostasis valve assembly 200. The flushing tube 400 may be utilized to introduce fluid into and through the delivery device 10, for example to purge air out of the delivery device 10 prior to use. Flushing tubes are generally well known as they pertain to delivery devices, and thus flushing tube 400 is not described in greater detail herein.

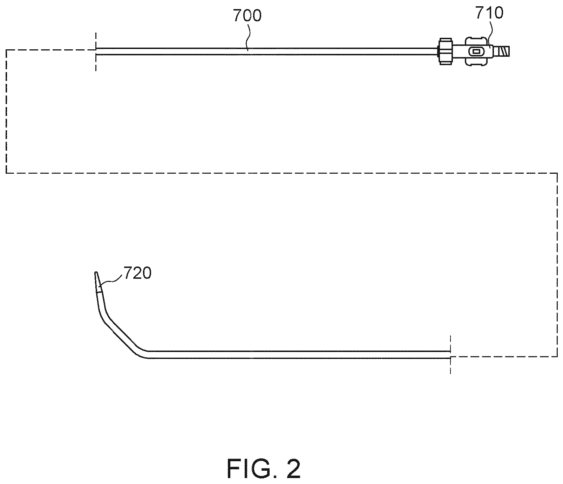

Fig. 2 illustrates a dilator 700 that may be used with delivery device 10. In the illustrated example, dilator 700 is a solid member that includes a connector 710 such as a luer lock at a proximal end thereof, and an atraumatic tip 720 at a distal end thereof. Although referred to as a "solid member," it should be understood that dilator 700 may include a guidewire lumen passing therethrough to allow for the dilator 700 to ride over a guidewire. In other words, dilator 700 may also be thought of as a "substantially solid" or thick-walled device. The distal end portion of the dilator 700 may, in the absence of applied forces, have an angle of about 45 degrees relative to the central longitudinal axis of the dilator 700. In other words, the dilator 700 may include a distal portion that has a neutral angle that is about the same as the neutral angle of the distal tip of the catheter 600, whether that angle is about 45 degrees or another value. The dilator 700 may have an outer diameter that is about equal to (or slightly smaller than) the inner diameter of the catheter 600. As will be described in greater detail below, the dilator 700 may be positioned within and through the catheter 600 during delivery of the delivery device 10 to the desired anatomical location. Then the dilator 700 may be removed to allow for other devices, such as an LAA occluder, to be passed into and through the delivery device 10 for implantation. In some embodiments, the distal portion of the dilator 700 may have curvature, or multiple angles generally similar to that shown in Fig. 2. As described in greater detail below, one concern with devices like delivery device 10 is that, if the hemostasis valve (such as hemostasis valve 240) within the delivery device tears or is otherwise damaged (e.g., from a device passing through the hemostasis valve), fluid (including air) may be more prone to leaking into or out of the delivery device. If the distal portion of the dilator 700 has curvature or multiple angled portions, there may be an increased risk that the dilator 700 tears the hemostasis valve as it passes through the hemostasis valve. Thus, in an alternate embodiment to the dilator 700 shown in Fig. 2, in other embodiments, the dilator may be completely straight (e.g., without any angles or curvature), or the distal portion may have an entirely straight portion that is at an angle relative to the main body of the dilator 700 *(e.g.* a single angled portion instead of a multi-angled portion as shown in Fig. 2). These other embodiments may allow for gentler interaction with the hemostasis valve as the dilator 700 is advanced through the hemostasis valve.

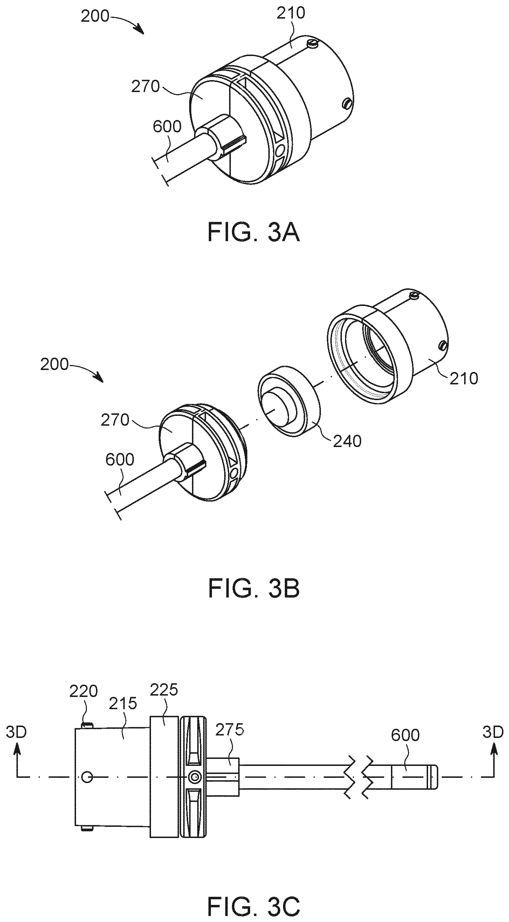

Fig. 3A shows hemostasis valve assembly 200. In the assembled condition, a cap 210 of the hemostasis valve assembly is coupled to a hub 270 of the hemostasis valve assembly, and a proximal end of the catheter 600 is coupled to a distal end of the hub 270. Although not shown in Fig. 3A, the handle 100 may be coupled to and extend from a distal end of the hub 270. Fig. 3B shows hemostasis valve assembly 200 in an exploded view, showing that the hemostasis valve 240 provides a seal between the cap 210 and the hub 270 in the assembled condition of the hemostasis valve assembly 200.

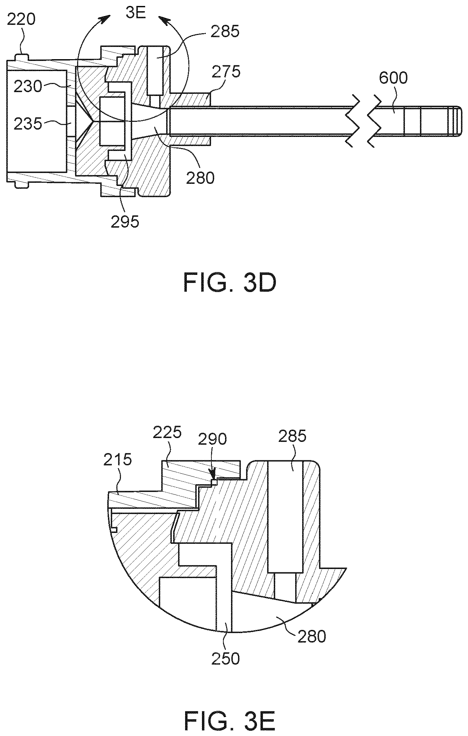

Referring to Figs. 3C-D, the proximal end of the catheter 600 may be received within and coupled to an extension 275 *(e.g.* a cylindrical extension) extending distally from a center of the hub 270. The interior of the cylindrical extension may be open such that, in the assembled condition, the inner lumen of catheter 600 is accessible from a proximal end of hub 270, via hemostasis valve 240, as described in greater detail below. The hub 270 may form a central aperture 280, which in the illustrated embodiment, is tapered from a relatively large proximal diameter, to a relatively small distal diameter where the central aperture 280 opens to the interior of the extension 275 and to the inner lumen of the catheter 600. The hub 270 may also define a flush port 285 that has a first end open to the exterior of the hub 270, and a second opposite end that opens to the central aperture 280. The flush port 285 is configured to couple to flushing tube 400, so that fluid pushed through the flushing tube 400 enters the hub 270 distal to the hemostasis valve 240, allowing for flushing and/or de-airing of the interior of the delivery device 10. Referring to Figs. 3D-E, the hub 270 may also include reduced outer diameter portions 290, which may be provided as stepped down diameters that form shoulders, extending proximally. With this configuration, correspondingly sized and/or shaped distal portions of the cap 210 may be coupled to the hub 270 at those locations, for example via ultrasonic welding, with the resulting assembly having a generally smooth outer diameter between the transition from the cap 210 to the hub 270. Lastly, the hub 270 may define a generally cylindrical recess 295 at a proximal end thereof, for example radially inwardly of the stepped portions 290 and proximal to the central aperture 280. This recess 295 may be sized and shaped to receive a distal portion of the hemostasis valve 240 therein in the assembled condition of the hemostasis valve assembly 200.

Referring now to Figs. 3A-D, cap 210 may include a main body 215 at its proximal end, which may be generally cylindrical and hollow. The main body 215 may include one or more protrusions 220 extending radially outward therefrom for interaction with the hemostasis valve knob 300, described in greater detail below. In the illustrated embodiment, each protrusion 220 is a cylindrical boss, and a total of four bosses are provided at about 90 degree spacing around the outer circumference of the main body 215. However, in other embodiments, more or fewer protrusions 220 may be provided, at the same or different relative spacing, and with shapes that are similar to or different than cylindrical bosses. At its distal end, the cap 210 may transition from main body 215 to a rim 225 having a diameter that is larger than the main body 215. The interior diameter of the cap 210 at the rim 225 may also be larger than the interior diameter at the main body 215. As shown in Figs. 3D-E, the interior surface of the rim 225 may include stepped portions that form shoulders that have a shape and configuration generally complementary to the stepped portions 290 of hub 270. As described above, these complementary features may assist in fixing the hub 270 to the cap 210, for example via ultrasonic welding, although other modalities (*e.g.* adhesives) may be suitable for the fixation.

Referring to Fig. 3D, the cap 210 may include an interior flange 230 extending radially inwardly from the main body 215, about halfway along the length of the main body 215. The interior flange 230 may define a substantially circular aperture 235 at or near a radial center of the cap 210, such that the aperture 235 is substantially coaxial with aperture 280 and catheter 600. With this configuration, a generally cylindrical recess may be formed, the recess having an open proximal end, and being bounded by the main body 215 and, at its distal end, the interior flange 230.

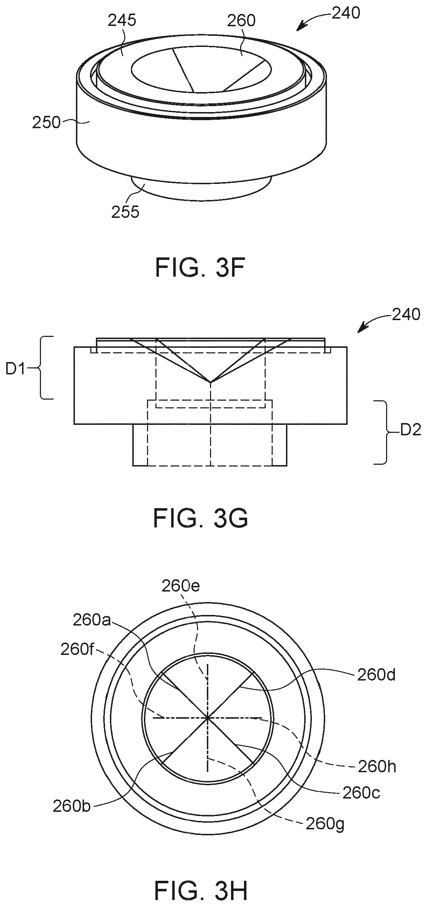

Referring now to Figs. 3B, 3D, and 3F, the hemostasis valve assembly 200 may include a hemostasis valve 240 positioned therein. As best shown in Fig. 3F, the hemostasis valve 240 may include a proximal section 245, a flanged section 250, and a distal section 255. The proximal section 245 may include a generally conical recess extending in a direction toward the distal section 255, the contours of which may assist in guiding a device into and through the hemostasis valve 240. Each of these three valve sections may have a generally circular or cylindrical shape (which may or may not include a taper), with the flanged section 250 having a larger outer diameter than the proximal section 245 and the distal section 255, and the distal section 255 having a smaller outer diameter than the proximal section 245.

The hemostasis valve 240 is preferably formed as a single integral member, and one or more cuts or slits are formed therein to create the actual valve functionality. One particular way of creating the valve functionality is described directly below, but it should be understood that other methodologies and other resulting valve structures may be suitable for use instead of the particular example shown and described herein. For example, Figs. 3G-H are side and top views, respectively, of the hemostasis valve 240 with slits made therein illustrated. In this particular example, four slits are formed in the proximal section 245 extending toward the distal section, and four slits are formed in the distal section 255 extending toward the proximal section, each group of four slits being formed in an "X" configuration at a spacing of about 90 degrees between adjacent slits, with the two groups of slits being offset rotationally from each other by about 45 degrees.

In particular, referring to Figs. 3G-H, four slits 260a-d are formed in the proximal section 240, each slit 260a-d extending a depth D1 toward the distal section 255. Each slit 260a-d is spaced about 90 degrees from an adjacent slit to form the cross or "X"-shape shown. These slits 260a-d may be thought of as forming flaps 260, labeled in Fig. 3F, having generally triangular or wedge shapes. The depth D1 may extend a depth into the flange section 250, but stop short of the distal section 255. The four slits 260a-d intersect at a central intersection point that extends a distance or depth to form a line where the slits intersect. A second group of slits 260e-h are formed in the distal section 255 extending a depth D2 toward the proximal section 245, having substantially the same configuration as slits 260a-d, except being offset, for example by about 45 degrees, relative to slits 260a-d. In particular, as shown in Fig. 3H, the four slits 260e-h form a cross or "X"-shape, with each slit spaced about 90 degrees from an adjacent slit in the group, and the four slits 260e-h meeting at a central intersection point that extends a distance of depth to form a line where the slits intersect. The pathway between the proximal section 245 and the distal section 255 is completed by the two intersection lines of the two groups of slits 260a-d, 260e-h both overlapping for the small distance by which depths D1 and D2 overlap, as shown in Fig. 3G.

When the hemostasis valve assembly 200 is assembled, the outer circumference of the flanged section 250 may be in contact with an inner surface of the main body 215 of the cap 210, just distal to the interior flange 230. The proximal section 245 of the hemostasis valve 240 may be in contact with a distal surface of the interior flange 230, with the center of the hemostasis valve 240, where the flaps 260 converge, substantially coaxial with the circular aperture 235 defined by the interior flange 230, as best shown in Fig. 3D. The distal section 255 of the hemostasis valve 240 may extend into the cylindrical recess 295 of the hub 270. As best shown in Fig. 3D, the distal section 255 may include an outer rim that is substantially coaxial with the central aperture 280 of the hub 270.

When the hemostasis valve assembly 200 is assembled, the connection between cap 210 and hub 270 is fluid-tight such that, in order for any fluid (or other objects) to pass into the cap 210 and through the hub 270 to the catheter 600, the fluid must pass through hemostasis valve 240. In the absence of applied forces, the flaps 260 of the hemostasis valve 240 create a fluid-tight seal so that fluid is prevented from passing through the hemostasis valve 240. It should be understood that hemostasis valves that have other specific configurations than that shown may be suitable for use with the hemostasis valve assembly 200.

Typically, hemostasis valves such as hemostasis valve 240 have a soft durometer, for example from about 20 - 70 Shore A durometer, and are typically formed of silicone and/or urethane and/or other similar materials. If a hemostasis valve is intended to allow a relatively large device to pass therethrough, the hemostasis valve will typically require a relatively large diameter and/or a relatively large thickness. As hemostasis valves get larger and/or thicker, it may require more force to push a device through the seal, for example because the seal may provide greater resistance against such passage. Also, at least partially because of the low or soft durometer of the material forming a hemostasis valve, one or more drops of silicone oil (or other lubricant) are typically provided by the valve manufacturer in the slits to help ensure that the flaps do not stick together, particularly if the valve is sitting on a shelf for a period of time between manufacture and use. The lubricant may be applied directly to the material of the valve or in other embodiments the lubricant may infuse or self-leach into the material. The requirement for a device to have a relatively large column force to easily pass through a hemostasis valve, as well as the possible contamination of that device with pre-applied or infused silicone oil (or another lubricant) as it passes through the valve, may be generally undesirable features, depending on the particular device being passed through the seal. The hemostasis valve assembly 200 described above, in combination with the hemostasis valve knob 300 described below, may overcome one or both of these possible undesirable features.

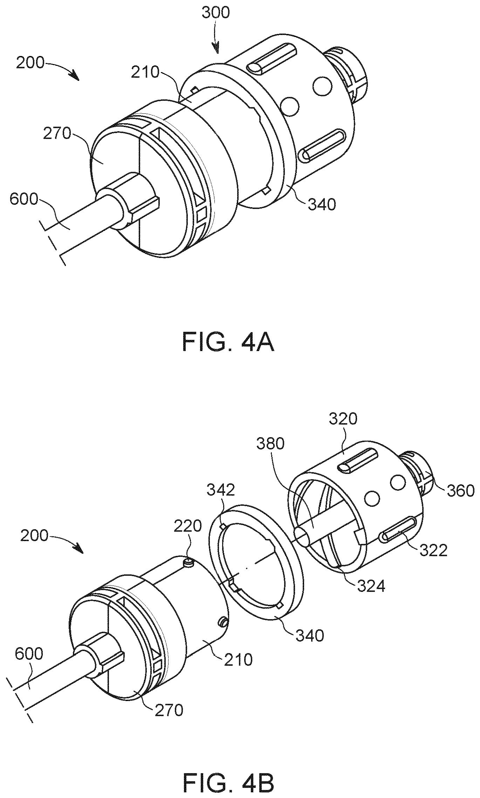

Fig. 4A shows the hemostasis valve assembly 200 assembled to the hemostasis valve knob 300, with Fig. 4B showing a corresponding exploded view. The hemostasis valve knob 300 may include a main body 320, a retaining ring 340, a bypass hub 360, and a bypass tube 380.

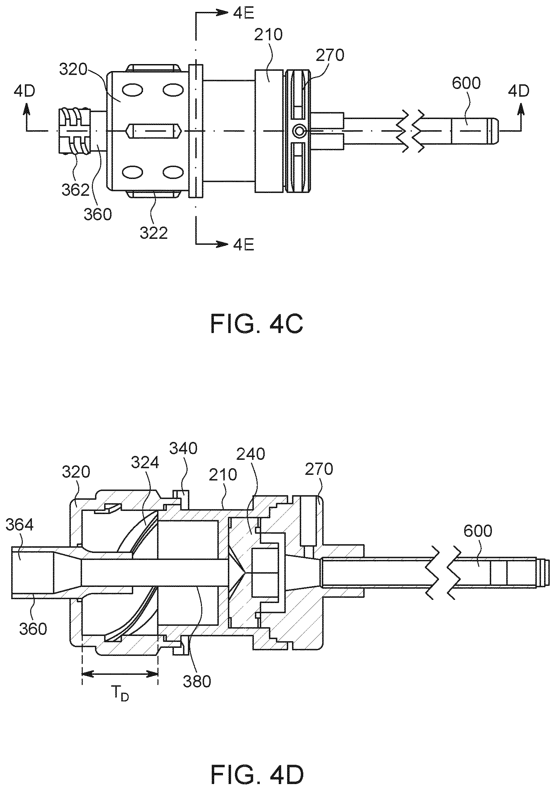

Referring generally to Figs. 4A-D, the main body 320 may be generally cylindrical and may include texturization on an outer surface to enhance a user's grip on the main body 320. In the illustrated example, the main body 320 includes four raised knurls 322 at equal circumferential spacing to assist a user in torquing the main body 320. However, it should be understood that other numbers, types, and spacing of texturization features may be provided instead of the raised knurls 322. The main body 320 may have a substantially open distal end, and an inner diameter that is sized to fit over the outer diameter of the main body 215 of cap 210. As best shown in Figs. 4B and 4D, the inner surface of main body 320 may include a plurality of curved channels or recesses 324, for example each in a generally helical configuration. Each curved recess 324 may extend to the terminal distal end of the main body 320, and may have a width and a depth sized to receive a corresponding protrusion 220 therein. With this configuration, when the main body 320 is assembled over the main body 215, and each protrusion 220 is received within a corresponding curved recess 324, rotating the main body 320 will translate the hemostasis valve knob 300 toward or away from the hub 270 of the hemostasis valve assembly 200, as described in greater detail below. For example, as shown in Fig. 4D, rotation of the main body 320 allows for distal translation or advancement of the main body 320 (along with the bypass hub 360 and bypass tube 380) a maximum available travel distance T_{D}, until the interior proximal face of the main body 320 contacts that proximal end of the cap 210. Although not shown in the drawings, in some embodiments, an O-ring, gasket, or other seal may be provided on the proximal end of the cap 210 so that, when the interior proximal face of the main body 320 is advanced fully, the O-ring, gasket, or other seal is compressed between the main body 320 and the cap 210, thereby sealing against fluid (including air) passing between the cap 210 and main body 320 into the interior space of the assembly. The actual available travel distance may be smaller, depending on the axial length of the recesses 324, for example. Although four curved recesses 324 are shown, more or fewer may be provided, preferably with equal number and spacing as the protrusions 220. And although referred to as a knob that is rotatable, the hemostasis valve knob 300 may also be referred to as an actuator that activates by rotation or other non-rotational movements.

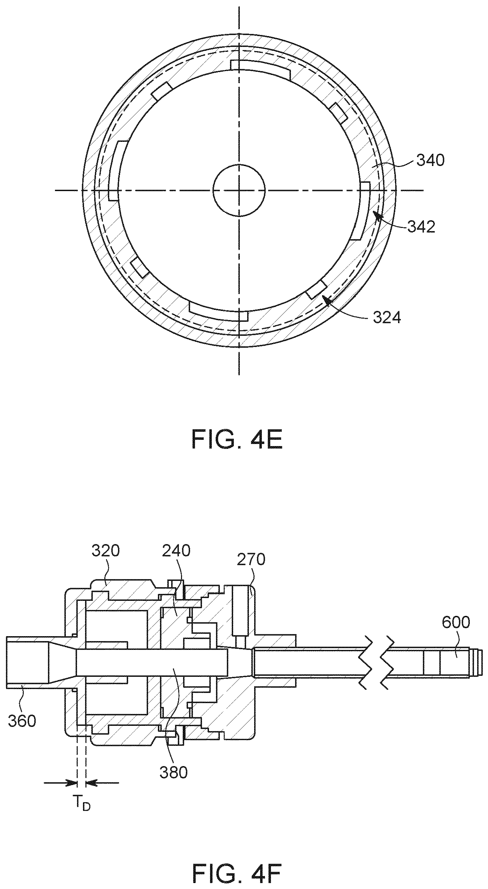

Retaining ring 340 may be a generally annular member that is sized to mate with the terminal distal surface of the main body 320. In particular, the retaining ring 340 may have a distal face with an inner diameter that is slightly smaller than the outer diameter of the main body 320, and an outer side wall that has an inner diameter that is about equal to or slightly larger than the outer diameter of the main body 320. As shown in Fig. 4D, this size configuration allows the retaining ring 340 to snap over the terminal distal end of the main body 320. As best illustrated in Figs. 4A-B, the retaining ring 340 may include a plurality of recesses 342 in the distal face thereof, preferably in the same number and relative spacing as protrusions 220 and curved recesses 340. The recesses 342 are sized and spaced so that, when each recess aligns with a corresponding protrusion 220, the retaining ring 340 may slide axially over the main body 215 of cap 210. However, if the recesses 342 are not aligned with corresponding protrusions 220, there is not enough clearance for the retaining ring 340 to slide axially past the protrusions 220. This configuration may help with assembling the main body 320 to the main body 215, with the retaining ring 340 ensuring that the main body 320 cannot disconnect from the main body 215. For example, during assembly, the retaining ring 340 may be oriented with recesses 342 aligned with protrusions 220 and slid distally over the main body 215. Then, the retaining ring 340 may be rotated, for example about 45 degrees, so that the recesses 342 no longer align with the protrusions 220. Then, the main body 320 may be coupled to the main body 215 with the protrusions 220 received within curved channels 324. The main body 320 may then be fixed to the retaining ring 340, such that the terminal distal ends of the curved recesses 324 are out of alignment with the recesses 342 of the retaining ring 340. The method for fixing may be any suitable method, including adhesives, ultrasonic welding, *etc.* With this configuration, the retaining ring 340 prevents the main body 320 from slipping off the main body 215 as it moves proximally away from the main body 215 upon rotation. Fig. 4E illustrates the coupling of the retaining ring 340 to the main body 320, with other components omitted for clarity. As can be seen, the recesses 342 of the retaining ring 340 are out of alignment with the ends of the curved recesses 324 in the main body 320.

Referring to Figs. 4C-D, the hemostasis valve knob 300 includes a bypass hub 360 and a bypass tube 380 extending through a proximal surface of the main body 320. The bypass hub 360 may have a generally cylindrical outer surface, and may include threads 362 or another mechanism to facilitate coupling to other devices used in conjunction with delivery device 10. The bypass hub 360 may be formed integrally with, or formed separately and then coupled to, the main body 320. The bypass hub 360 may define a lumen 364 therethrough, and the lumen 364 may be tapered in the distal direction. The lumen 364 is preferably coaxial with the other lumens and openings within the hemostasis valve assembly 200 such as aperture 235, aperture 280, the overlapping openings of hemostasis valve 240, and is also preferably coaxial with the catheter 600.

Referring now to Fig. 4D, the bypass tube 380 extends distally from the bypass hub 360. The bypass tube 380 is preferably generally cylindrical with an outer diameter that is about equal to or just smaller than the interior diameter of aperture 235. The bypass tube 380 may be formed integrally with the bypass hub 360, for example via injection molding, in which case suitable materials may include acrylonitrile butadiene styrene ("ABS"). In other embodiments, the bypass tube 380 may be formed of materials such as polyoxymethylene (*e.g*. under the tradename Delrin^{®}), etched polytetrafluoroethylene, polyether block amide (*e.g.* under the tradename Pebax^{®}), or other suitable materials such as Nylon (*e.g.* lined Nylon 12 tubing). The bypass tube 380 preferably has a relatively high column strength, such that it may easily pass through hemostasis valve 240 without buckling or otherwise being damaged as it translates distally. For example, the bypass tube 380 may have a column strength that is greater than the column strength of the medical device that is to be passed through the bypass tube. The bypass tube 380 preferably has a length so that, when the main body 320 is in its proximalmost position relative to the cap 210, the distalmost end of the bypass tube 380 is positioned within aperture 235 just proximal of the hemostasis valve 240. Although not shown, in some embodiments, an O-ring, gasket, or other seal may be provided on the interior surface that defines the aperture 235, so that a sealing member is always compressed or otherwise positioned between the outer surface of the bypass tube 380 and the inner surface that defines aperture 235. This seal, if present, may additionally help ensure that fluid (including air) is not capable of passing between the outer surface of the bypass tube 380 and the inner surface that defines aperture 235.

It should be understood that the hemostasis valve assembly 200 and knob 300 are illustrated in Figs. 4A, 4C, and 4D in a sealed or first condition, in which the main body 320 is in its proximalmost position relative to the cap 210. In this condition, as noted above, the distalmost end of the bypass tube 380 is positioned just proximal to the hemostasis valve 240, so that the bypass tube 380 does not traverse the hemostasis valve 240. A user may transition the hemostasis valve assembly 200 and knob 300 into an open or second condition by rotating the main body 320 relative to the cap 210, forcing the hemostasis valve knob 300 to translate distally until the bypass tube 380 passes through the hemostasis valve 240. This open or second condition is illustrated in Fig. 4F. As shown in Fig. 4F, the main body 320 of the hemostasis valve knob 300 has been rotated to advance the main body 320, as well as the retaining ring 340, bypass hub 360, and bypass tube 380 distally relative to the hemostasis valve assembly 200. The completion of the advancement can be seen by, for example, comparing the available travel distance T_{D} shown in Fig. 4D having been decreased to substantially zero, with the inner face of the proximal main body 320 abutting the proximal end of cap 210. In this second or open condition, the bypass tube 380 fully traverses the hemostasis valve 240, with the distal terminal end of the bypass tube 380 positioned within or adjacent to central aperture 280. Thus, in the first or sealed condition shown in Fig. 4D, the hemostasis valve 240 is closed and the lumens or openings distal to the hemostasis valve 240 are sealed from the lumens or openings proximal to the hemostasis valve 240. However, in the second or open condition shown in Fig.4F, the bypass tube 380 forces the hemostasis valve 240 to remain open, putting the lumens or openings distal of the hemostasis valve 240 in fluid communication with the lumens or openings proximal to the hemostasis valve 240.

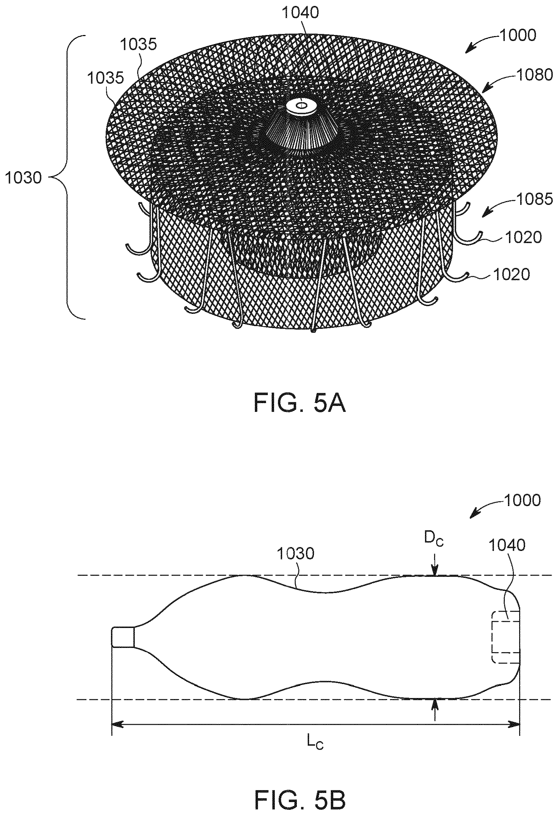

Fig. 5A is a perspective view of an occluder 1000, which may be a LAA occluder. Very generally, occluder 1000 includes an occluding material forming a tubular structure 1030, the occluding material being a braided metal fabric, which may be formed by braiding a plurality of strands 1035, which may be strands of nickel titanium alloy *(e.g.* under the tradename Nitinol), together and shape set *(e.g.* via heat setting) to the shape shown in Fig. 5A. When in the shape-set or expanded condition (e.g. in the absence of applied forces), the occluder 1000 may include a generally disk-shaped portion 1080 configured to abut an ostium of the patient's LAA, and a second generally cylindrical portion 1085 configured to be received within the patient's LAA. In some embodiments, the disk-shaped portion 1080 may be coupled to the cylindrical portion 1085 via a small diameter transition portion. The cylindrical portion 1085 may include one or more hooks 1020 configured to engage tissue within the LAA upon deployment. The disk-shaped portion 1080 may include a connector 1040, which may for example include internal threads, for coupling to a delivery cable 1300.

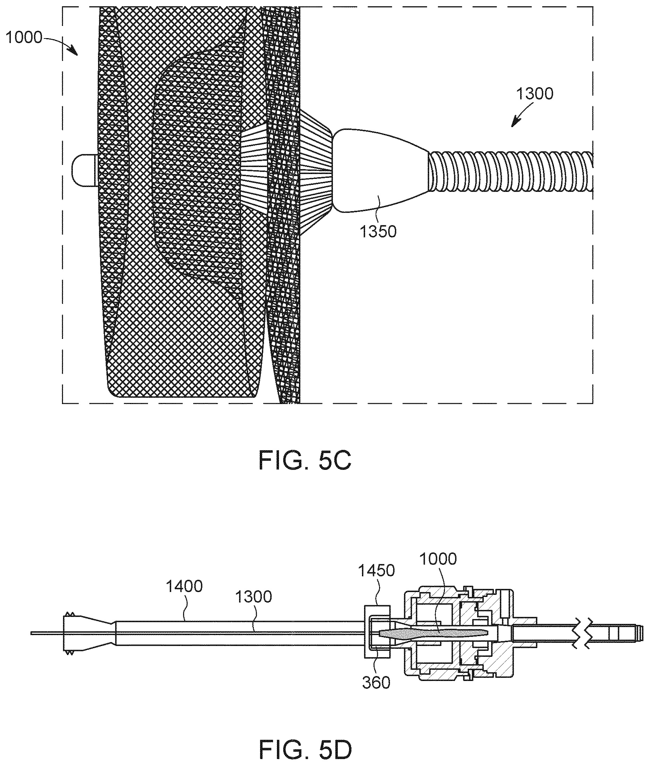

Fig. 5B illustrates occluder 1000 in a collapsed condition, having a collapsed diameter D_{C} smaller than the diameter of the disk-shaped portion 1080 and the cylindrical portion 1085 when in the expanded condition shown in Fig. 5A, and a collapsed length L_{C} longer than the length of the occluder in the expanded condition shown in Fig. 5A. Fig. 5C illustrates the delivery cable 1300 coupled to the connector 1040, for example via a threaded hub 1350 of the delivery cable 1300 that has been threaded into the connector 1040.

An exemplary use of the delivery device 10 with the integrated hemostasis bypass valve for delivering occluder 1000 is described below. At the beginning of the procedure (or in preparation thereof), the delivery device 10, including dilator 700, may be removed from sterile packaging. The user preferably confirms that the hemostasis valve knob 300 is in the first, sealed position such that the hemostasis valve 200 is closed. If the hemostasis valve knob needs to be adjusted, the user may rotate the hemostasis valve knob 300, for example by turning it counter-clockwise relative to the hemostasis valve assembly 200, to retract the bypass tube 380 so that it does not pass through the hemostasis valve 240. The catheter 600 and the dilator 700 may be wiped with sterile gauze dampened with sterile saline to remove any foreign material that may be on the components. The user may then pass the dilator 700 through the delivery device 10 until the distal end of the dilator 700 passes the distal end of the catheter 600. Even though the hemostasis valve knob 300 is in the first, sealed position, the dilator 700 has enough column strength to readily pass through the hemostasis valve 240 without assistance of the bypass tube 380, without any buckling or damage occurring to the dilator 700. When the dilator 700 is fully inserted through the delivery device 10, the connector 710 (or a threaded collar associated with the connector 710) may be rotated to couple to the bypass hub 360, for example by inner threads of the connector 710 engaging outer threads 362 of the bypass hub. The dilator 700 is now coupled to the remaining portions of the delivery device 10 as a single unit. Access may be gained to the patient via any suitable method, and a guidewire may be advanced into the patient's vasculature until it reaches the patient's left atrium, LAA, or pulmonary vein. Prior to or during introduction of the guidewire, a puncture may be made through the patient's atrial septum if the delivery route is, for example, through the patient's femoral vein and to the right atrium via the inferior vena cava, with the septal puncture allowing the guidewire and other components to traverse the atrial septum into the left atrium. With the guidewire in place, the dilator 700 (and the remainder of the delivery device 10 to which it is coupled) may be advanced over the guidewire into the patient until the distal end of the delivery device 10 reaches the left atrium or LAA. With the distal end of the delivery device 10 in the desired location, the connector 710 may be rotated to decouple it from the bypass hub 360, and the dilator 700 may be withdrawn from the catheter 600, preferably slowly to prevent any ingress of air. After the dilator 700 is fully removed, the guidewire may next be fully removed from the delivery device 10 and the patient, although the guidewire may instead be simultaneously removed with the dilator 700.

With the distal end of the catheter 600 in the desired position, the occluder 1000 may be introduced into and through the delivery device 10. As shown in Fig. 5D, a loading tube 1400 provided with the occluder 1000 may be coupled to the bypass hub 360, for example by rotating a threaded connector 1450 (or a threaded collar associated therewith) onto the bypass hub 360. The occluder 1000 may be pushed through the loading tube 1400 toward the hemostasis valve assembly 200 while the occluder 1000 is in the collapsed condition, by pushing delivery cable 1300. Prior to the occluder 1000 reaching the hemostasis valve 240, the user rotates the hemostasis valve knob 300, for example in clockwise direction, so that the bypass tube 380 advances into and through the hemostasis valve 240. However, in some embodiments, it may be appropriate to activate the bypass mechanism to get a wet-to-wet connection with the loading tube 1400 prior to the occluder 1000 reaching the hemostasis valve 240. Either way, with the hemostasis valve knob 300 in the second, open condition, the user may push the cable 1300 to advance the collapsed occluder through the bypass tube 380, bypassing the need for the occluder 1000 to contact the hemostasis valve 240 directly as the occluder 1000 passes through the hemostasis valve assembly 200. At any point after the occluder 1000 has advanced distally beyond the hemostasis valve 240, and prior to the occluder 1000 being deployed from the catheter 600, the hemostasis valve knob 300 may again be rotated to revert the hemostasis valve knob 300 into the first, closed condition. The cable 1300 preferably is able to readily pass through the closed hemostasis valve 240 without the assistance of the bypass tube 380. The user may continue pushing the delivery cable 1300 until the occluder reaches the distal end of the catheter 600, and then deploy the occluder 1000 from the distal end of the catheter 600, allowing the occluder 1000 to self-expand into the LAA to occlude the LAA. This process may be coupled with steering of the catheter 600 using the handle 100 and deflection knob 500, if steering capabilities are included in the delivery device 10. With the occluder 1000 deployed in the desired position, the cable 1300 may be decoupled from the occluder 1000, for example via rotation of the cable 1300, and the cable 1300 may be withdrawn from the delivery device 10.

As described above, there are at least two benefits that may be provided by the hemostasis valve bypass components. First, various medical devices that are collapsible for delivery through a catheter may have low column strength, especially compared to components like dilator 700. Occluder 1000 is formed of a braided mesh of strands of nickel titanium alloy, which may result in the occluder 1000 having a low column strength. Without the bypass tube 280, as the user pushed the occluder 1000 through the hemostasis valve 240, the occluder 1000 might buckle and become damaged as it encounters resistance from the hemostasis valve 240. Further, if the hemostasis valve 240 has silicone oil or another lubricant from when it was manufactured, that silicone oil may transfer to the occluder 1000, effectively contaminating the occluder with that substance. The bypass tube 280 solves both of these problems, by eliminating resistance that the occluder 1000 would encounter from the hemostasis valve 240, as well as ensuring no transfer of contaminants occurs since there is no direct contact between the occluder 1000 and the hemostasis valve 240. Further, the embodiments disclosed herein allow for the hemostasis valve assembly 200 and hemostasis valve knob 300 to be integrated with the remainder of the delivery device 10. In other words, the functionality of the bypass tube 380 is provided without the need for another separate device beyond the delivery device 10, increasing the convenience and decreasing the procedure time that would be required by having the bypass tube 380 a fully separate component.

Although the integrated hemostasis bypass valve described herein may be particularly useful for the LAA occluder 1000 device described herein, it should be understood that this particular use is merely exemplary. For example, the integrated hemostasis bypass valve described herein may be particularly useful for other occluders formed of braided metal, including septal occluders, patent ductus arteriosus ("PDA") occluder devices, patent foramen ovale ("PFO") closure devices, *etc.* It should further be understood that the integrated hemostasis bypass valve described herein may work well with other occluders, including those not formed from a braided mesh, or any other medical device, whether an occluder or not and whether formed of braided mesh or not, if that medical device has relatively low column strength and/or if it would be undesirable for that medical device to be contaminated with silicone oil or another lubricant from direct contact with a hemostasis valve.

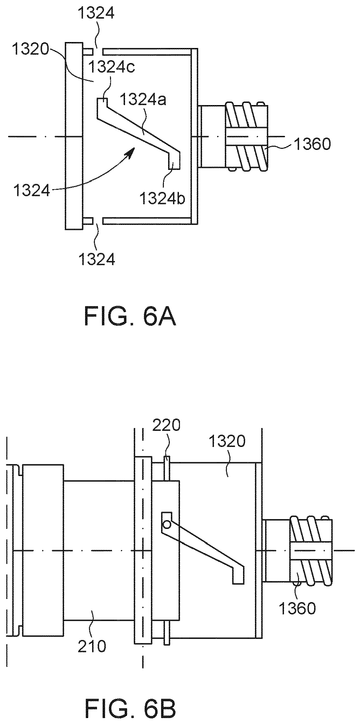

Although specific embodiments have been described above, additional variations are possible. For example, the embodiment above included a hemostasis valve knob 300 having a main body 320, retaining ring 340, bypass hub 360, and bypass tube 380. Some of these components may be further modified or altered to achieve desirable functionality. In one particular example, shown in Figs. 6A-B, an alternate version of main body 1320 of the hemostasis valve knob 300 is provided. It should be understood that all other components of this version of the hemostasis valve knob that are not described in detail here may be similar or identical to those described in connection with hemostasis valve knob 300. Similarly, any functionality of this version of the hemostasis valve knob that is not described in detail may be similar or identical to the functionality (including interaction with other components of the system) of that described in connection with hemostasis valve knob 300.

Fig. 6A is a side view of main body 1320, which may be similar or identical to main body 320 with the main exception being the inclusion of one or more enclosed slots 1324 instead of the curved recesses 324 described in connection with main body 320. As with main body 320, main body 1320 may be generally cylindrical and may include texturization on an outer surface to enhance a user's grip on the main body 1320, such as raised knurls (not shown). The main body 1320 may have a substantially open distal end, and an inner diameter that is sized to fit over the outer diameter of the main body 215 of cap 210. As shown in Figs. 6A-B, main body 1320 may include a plurality helical, curved, or angled slots 1324 that are preferably closed on each end. These slots 1324 may be solely formed on an interior surface of the main body 1320, or may be formed to extend through the entire wall thickness of the main body 1320. Referring to Fig. 6A, each slot 1324 may include a central portion 1324a that extends in a proximal-to-distal direction at an angle that is oblique to the central longitudinal axis of the main body 1320. Although in the side view of Fig. 6A the central portion 1324a appears to extend in a perfectly linear angle, it should be understood that the central portion 1324a may be thought of as more helical as it curves around the circumference of the main body 1320. Each slot 1324 may include a proximal terminal end 1324b that extends a short distance in the circumferential direction of the main body 1320, and a distal terminal end 1324c that extends a short distance in the circumferential direction of the main body 1320. As should be clear from Figs. 6A-B, the proximal and distal terminal ends 1324b, 1324c preferably both extend in the circumferential direction (e.g. in a direction around the central longitudinal axis of the main body 1320), with each terminal end extending in opposite directions from central portion 1324a. In other words, the proximal and distal terminal ends 1324b, 1324c preferably do not have any axially component of extension. Further, the circumferential component of the directionality from proximal terminal end 1324b, through central portion 1324a, and to distal terminal end 1234c, is continuous without a change in direction of the circumferential component, and is bounded on each end by structure of the main body 1320 enclosing the slot 1324.

The configuration of slots 1324 described above may be in contrast with curved recesses 324, which may be unbounded on the distal terminal end. Because curved recesses 324 are unbounded at the distal terminal end, the retention ring 340 is provided to help ensure the main body cannot slip off the proximal end of cap 210. However, because the slots 1324 are enclosed, there is little or no risk of the main body 1320 slipping off the proximal end of cap 210. However, a retention ring may still be provided with main body 1320, if desired.

The hemostasis valve knob may include a bypass hub 1360 similar or identical to bypass hub 360, a bypass tube (not shown) similar or identical to bypass tube 380, and as noted above, may or may not include a retention ring. In use, however, the main body 1320 functions similarly to main body 320. Referring to Fig. 6B, when assembled, the protrusions 220 of cap 210 each extend into a corresponding slot 1324. With this configuration, rotating the main body 1320 in one direction relative to cap 210 advances the main body 1320 (and thus bypass tube), while rotation in the opposite direction retracts the main body 1320 (and thus bypass tube). The exclusion of any axial extent to the proximal and distal terminal ends 1324b, 1324c of the slot 1324 helps to lock the hemostasis valve knob either in the open or closed condition. For example, when the bypass tube is not passing through hemostasis valve 240, the protrusions sit within the distal terminal ends 1324c of the slot 1324, as shown in Fig. 6B. As the main body 1320 is rotated (clockwise in this example), the protrusions 220 exit the distal terminal end 1324c, and are then guided along the angled center portion 1324a, until the protrusions 220 are sitting within the proximal terminal end 1324b of the slot 1324. When the protrusions 220 are in the proximal terminal end 1324b of the slot 1324, the bypass tube is passing through the hemostasis valve 240 providing the bypass functionality in the same way described above for hemostasis valve knob 300. It should be understood that the protrusions 220 of the main body 215 may be formed integrally with the rest of the main body 215 *(e.g.* via injection molding) or may be provided as separate components (e.g. pins) that are mounted or coupled to the main body.

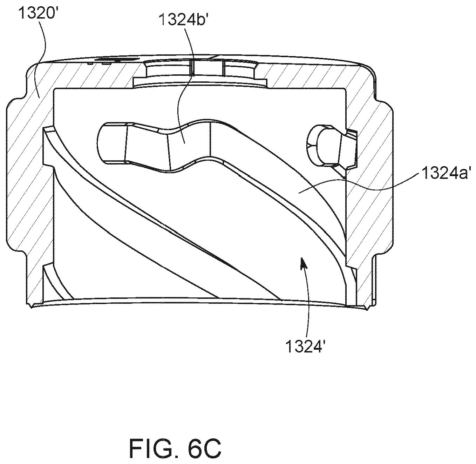

Fig. 6C shows a cutaway view of an alternate version of main body 1320' that is generally similar to main body 1320. Fig. 6C illustrates that each slot 1324' includes a generally helical central portion 1324a' bounded by a proximal terminal end 1324b' and a distal terminal end (not shown). One difference between main body 1320' and main body 1320 is that the proximal terminal end 1324b' includes a contoured section that forms a pocket that extends slightly distally. Whereas proximal terminal end 1324b has no axial extent, the slight axial extent of the contour of the proximal terminal end 1324b' may help to ensure that the protrusion 220, when sitting within the pocket of the contour, is not easily able to move out of that pocket without an intentional application of rotational force. The distal terminal end (not shown) may have a substantially identical contour as the proximal terminal end 1324b', although it may be mirrored in the sense that the contour of the distal terminal end may form a pocket that has a slight axial extension in the proximal direction. In addition or alternatively to the axial extents or pockets in the proximal terminal end 1324b' and the distal terminal end, divots extending radially inwardly may be formed in the proximal terminal end 1324b' and the distal terminal end. If such divots are included, the protrusions 220 may be formed to be retractable while being biased (e.g. spring biased) to an extended position. As the protrusions ride along the central portion 1324a', the protrusions may remain slightly retracted, but as the protrusions reach the divot of the proximal terminal end 1324b' or the distal terminal end, the protrusions 220 may extend into the divot, further locking the main body relative to the protrusions 220. If included, the action of the protrusions extending into the divots may also provide for audible and/or tactile feedback to confirm that the main body 1320' has reached its terminal extent of travel in the relevant direction. Otherwise, main body 1320' may function similarly or identically to main body 1320, including any variations described therewith.

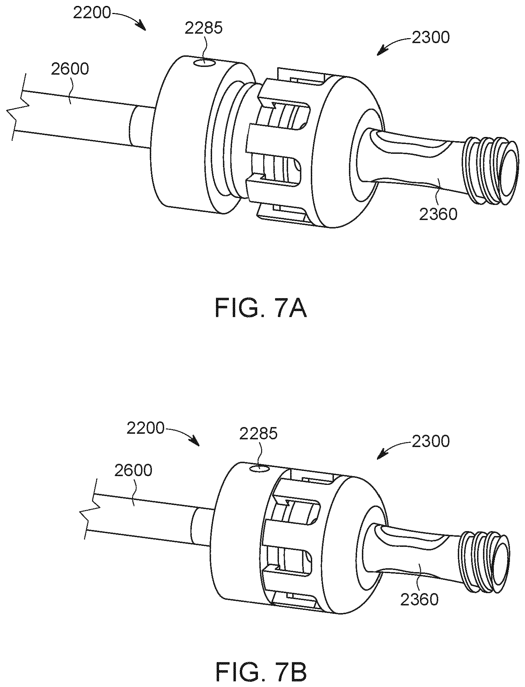

While main bodies 1320 and 1320' provide alternatives to main body 320, each of those specific examples of main bodies rely on a "twist-to-bypass" mechanism in which a hemostasis bypass knob is rotated in order to advance (or retract) the bypass tube and activate (or deactivate) the hemostasis valve bypass functionality. However, still other configurations may be suitable to achieve similar functionality, including a "push-to-bypass" mechanism. One example of a "push-to-bypass" mechanism is shown in Figs. 7A-D.

Referring to Figs. 7A-B, a hemostasis valve assembly 2200 is shown assembled to a hemostasis valve knob (or actuator) 2300. Hemostasis valve assembly 2200 and hemostasis valve knob 2300 may be used as part of a system like delivery device 10, in place of hemostasis valve assembly 200 and hemostasis valve knob 300, including any of the variants described therewith. For example, the delivery device utilizing hemostasis valve assembly 2200 and hemostasis valve knob 2300 may be used with a catheter sheath 2600 that is steerable or non-steerable. Although the term "knob" is used, it should be understood that the term knob does not require turning or rotation to activate, but includes handles or members that may be pushed to activate.

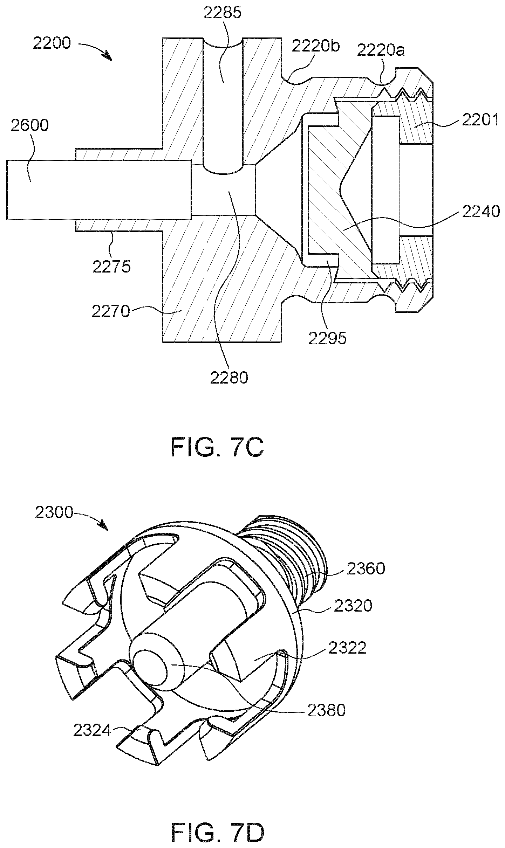

Fig. 7A illustrates the assembly with the hemostasis valve 2240 in a closed (or non-bypassed) condition, and Fig. 7B illustrates the assembly with the hemostasis valve 2240 in an open (or bypassed) condition. Fig. 7C is a cross-section of the hemostasis valve assembly 2200, and Fig. 7D is a perspective view of hemostasis valve knob 2300.

Referring to Fig. 7D, the hemostasis valve knob 2300 may include a main body 2320, a bypass hub 2360, and a bypass tube 2380. Bypass hub 2360 and bypass tube 2380 may be similar or identical to bypass hub 360 and bypass tube 380 (including variations described therewith), and are thus not described in detail again here. As should become clear, a retaining ring similar to retaining ring 340 may not be needed for hemostasis valve knob 230. The main body 2320 may include proximal portion coupled (which includes the possibility of being formed integrally with) the bypass hub 2360, and the proximal portion may begin to transition into a cylindrical housing that includes a plurality of locking arms 2322 extending distally therefrom. In the illustrated embodiment, the main body 2320 includes six locking arms 2322, although more or fewer, such as 4, 5, 7, 8, 9, 10, 11, or 12, may be provided. The locking arms 2322 may include outer surfaces that are all positioned along the surface of the same imaginary right cylinder. Each locking arm 2322 may be a thin walled distal extension of the main body 2320, with an inward protrusion or hook 2324 at the terminal distal end of each locking arm 2322. Each hook 2324 extends generally radially inwardly back toward the center longitudinal axis of the hemostasis valve knob 2300. The tip of each hook 2324 may have any desired shape, but in the illustrated embodiment the tips of each hook have a generally semicircular profile.

Referring now to Fig. 7C, the hemostasis valve assembly 2200 may include a hub 2270 that may include substantially the same internal configuration as hub 270, for example including a central aperture 2280 that leads to extension 2275 (which may couple to the proximal end of catheter sheath 2600). The hub 2270 may also include a flush port 2285 that leads to central aperture 2280 to allow for flushing downstream of (or distal to) the hemostasis valve 2240. The hub 2270 may define a recess 2295 (which may be cylindrical) that may be sized and shaped to receive a distal portion of the hemostasis valve 2240 therein in the assembled condition of the hemostasis valve assembly 2200. Hemostasis valve 2240 may be similar or identical to hemostasis valve 240, and is thus not described again in detail here.

One of the main differences between hub 2270 and hub 270 is that hub 2270 includes two sets of grooves on an outer surface thereof. In the illustrated example, a proximal groove 2220a is positioned a spaced distance from a distal groove 2220b. As best shown in Figs. 7A-B, each of the grooves 2220a, 2220b may extend around the entire circumference of the hub 2270, with each groove 2220a, 2220b having a generally semicircular profile (as best shown in Fig. 7C). In this configuration, the outer surface of hub 2270 may be raised relative to the grooves 2220a, 2220b just proximal to each groove.

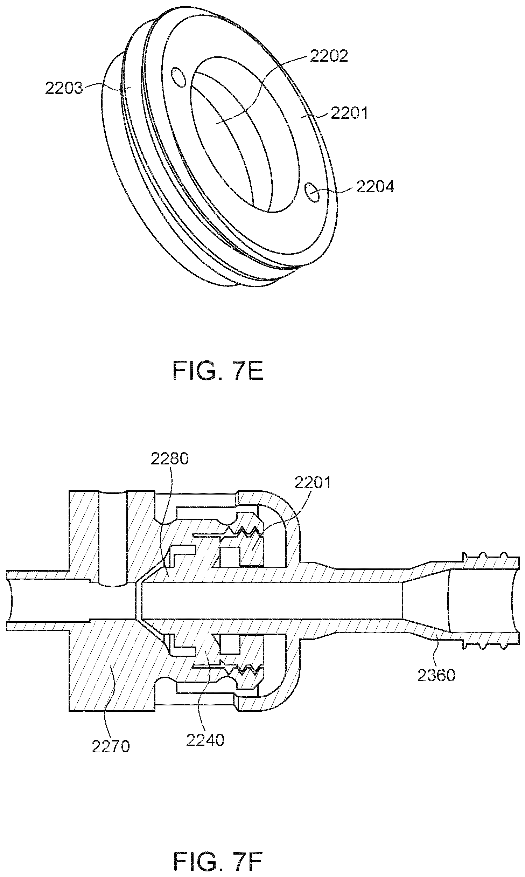

Still referring to Fig. 7C, a plug 2201 may be provided to help seal against bypass tube 2380. Plug 2201 is shown in more detail in Fig. 7E. Plug 2201 may have a generally annular shape and define in internal aperture 2202 having an internal diameter about equal to the outer diameter of bypass tube 2380, so that bypass tube 2380 can snugly pass through the center of plug 2201 while maintaining a seal. Referring back to Fig. 7C, the hemostasis valve 2240 may be sandwiched between plug 2201 and the internal components of hub 2270. In the illustrated embodiment, plug 2201 includes external threads 2203 to engage internal threads of the proximal end of the hub 2270 to couple the plug 2201 to the hub 2270. However, in other embodiments, threads 2203 may be omitted and the plug 2201 may be fixed in place by other mechanisms, such as ultrasonic welding, bonding, *etc.* If the plug 2201 is connected via threading, it may include torque features, such as apertures 2204, so that a tool may engage with the plug 2201 to thread it into the hub 2270.

In the non-bypassed condition of Fig. 7A, each of the hooks 2324 of each locking arm 2322 is received within the proximal groove 2220a. When in this condition, the distal end of the bypass tube 2380 is spaced proximally from the hemostasis valve 2240, allowing the hemostasis valve 2240 to be closed (unless another component is extending through the hemostasis valve 2240, such as an introducer). In order to activate the bypass functionality, the user need only push the hemostasis valve knob 2300 distally relative to the hemostasis valve assembly 2200. As the user applies distally directed force, the locking arms 2322 will begin to flex and the hooks 2324 will be driven out of the proximal groove 2220a, and slide along the outer surface of the hub 2270 until the hooks 2324 reach the distal groove 2220b. It should be understood that the locking arms 2322 are in a generally relaxed condition when the hooks 2324 are within either groove 2220a, 2220b, but are in a flexed condition when the hooks 2324 are in contact with the outer surface of the hub 2270 between the grooves 2220a, 2220b. As a result of this configuration, when the hooks 2324 reach distal groove 2220b, the locking arms 2322 will "snap" back to the relaxed condition in which the hooks 2324 are received within the distal groove 2220b. When the hooks 2324 are received within the distal groove 2220b, as shown in Fig. 7B, the bypass tube 2280 extends through the hemostasis valve 2240. In order to switch from the bypassed condition to the non-bypassed condition, the user may just pull the hemostasis valve knob 2300 proximally until the hooks 2324 of locking arms 2322 snap back into the proximal grove 2220a.

Fig. 7F is a cross-section of the hemostasis valve knob 2300 and hemostasis valve assembly 2200 in the bypassed condition, with the distal end of bypass tube 2280 having passed through hemostasis valve 2240. It should be understood that, in the particular view of Fig. 7F, the plane is taken between adjacent locking arms 2322 so that the locking arms 2322 (and particularly the hooks 2324) are not visible despite being located within distal groove 2220b.

It should be understood that certain modifications may be made without departing from the scope of the invention. For example, instead of having continuous grooves 2220a, 2220b, the outer surface of the hub 2270 could have two rows of individual detents or divots in number and spacing that correspond to the number and spacing of the locking arms 2322 and hooks 2324. Further, although semicircular profiles are shown for the grooves 2220a, 2220b and for the tips of hooks 2324, other shapes may be appropriate, particularly if they allow for the hooks 2324 to be manually pushed out of proximal groove 2220a, and manually pulled out of distal groove 2220b, while also allowing for a secure connection that is unlikely to result in a user unintentionally switching between the bypassed and non-bypassed condition.

Additional description of the use of hemostasis valve assembly 2200 and hemostasis valve knob 2300 is not provided herein because the description of the use of hemostasis valve assembly 200 and hemostasis valve knob 300 applies equally, with the exception that the "twist-to-bypass" functionality is replaced with the "push-to-bypass" functionality described above.

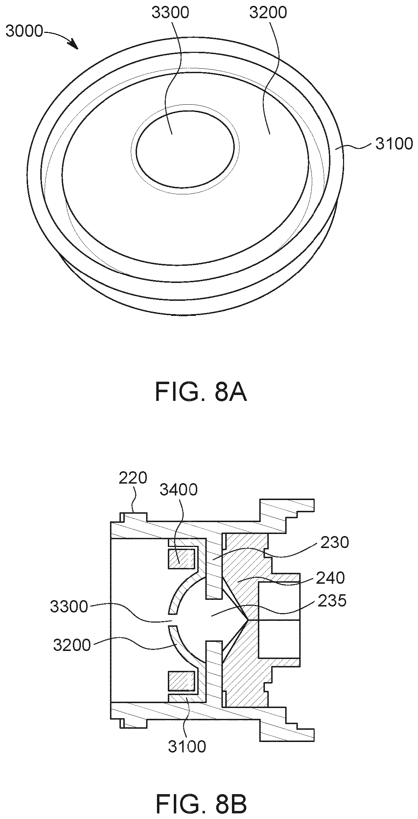

Although the various bypass configurations are described above in connection with a single seal (e.g. hemostasis valve 240 or 2240), in any of the embodiments described herein, one or more additional seals, such as wiper seals, may be provided. For example, Fig. 8A illustrates a wiper seal 3000 that may be used as an additional component in any of the systems described above. Wiper seal 3000 may take various configurations, but in the illustrated embodiment, wiper seal 3000 is generally circular with an outer rim 3100 that transitions to generally proximally contoured main body 3200 defining a central aperture 3300 that allows for devices to pass therethrough. Fig. 8B illustrates the wiper seal 3000 within cap 210 of hemostasis valve assembly 2200 (with hub 270 omitted from the figure), although as noted above wiper seal 3000 may be used with any embodiment described herein. In this embodiment, the distal end of wiper seal 3000 abuts the proximal end of flange 230, with the central aperture 3300 substantially coaxial with circular aperture 235. The wiper seal 3000 may be coupled to the interior of cap 210 by any suitable means, including for example UV bonding or ultrasonic welding a seal ring 3400 to the inner housing of cap 210. The sealing ring 3400 may include, for example, a raised boss to engage the wiper seal 3000 and help keep the wiper seal 3000 in a desired position. In use, as the bypass tube 380 is advanced from the non-bypass condition to the bypass condition, the outer surface of the bypass tube 380 slides against the interior surface of the wiper seal 3000 that defines aperture 3300, such that any contaminants or other substances deposited on the bypass tube 380 will be prevented from passing beyond the wiper seal 3000. In other words, the additional wiper seal 3000 may help limit any contaminants of other foreign matter on the outer surface of the bypass tube 380 from getting into and/or past the hemostasis valve 240 and potentially into the patient's body.

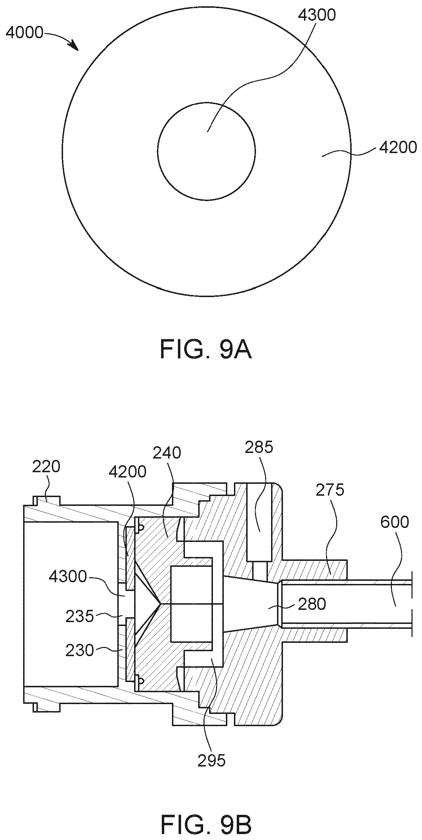

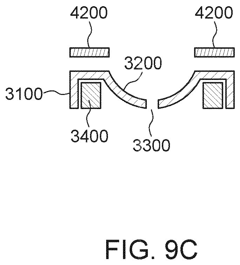

Fig. 9A is a front view of a flat wiper seal 4000 which may have an annular main body 4200 defining a central aperture 4300. In use, the flat wiper seal 4000 may be coupled to a distal face of interior flange 230, proximal to hemostasis valve 240, with the central aperture 4300 substantially coaxial with circular aperture 235 and the opening in hemostasis valve 240, as shown in Fig. 9B. The coupling may be achieved by any suitable mechanism, including for example UV bonding or ultrasonic welding. With this configuration, the wiper seal 4000 is effectively sandwiched between the interior flange and the hemostasis valve 240, but functions substantially similar to the functioning of wiper seal 3000 described above. As with wiper seal 3000, wiper seal 4000 may be implemented in any of the embodiments described above, and both wiper seal 3000 and wiper seal 4000 may be used together in a single system. For example, Fig. 9C illustrates both wiper seals 3000 and 4000 used together (with the remaining components of hemostasis valve assembly 200 omitted from the view for clarity).

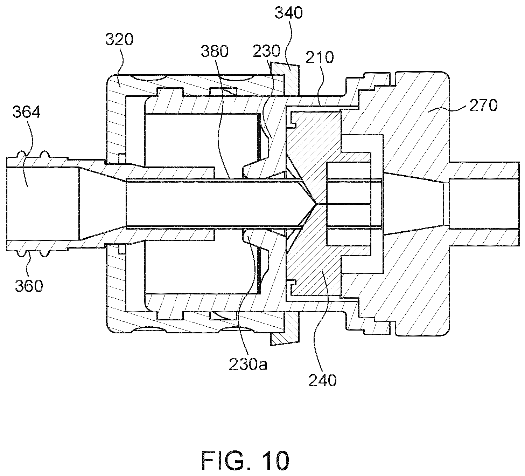

As noted above, air ingress across the hemostasis valve is undesirable during a procedure. One issue that can increase the risk of air ingress is if the hemostasis valve tears or is otherwise damaged, for example as a result of components being passed through the hemostasis valve. The bypass tube is one component which is at risk of tearing or damaging the hemostasis valve, particularly if the bypass tube does is not perfectly (or close to perfectly) coaxially aligned with the hemostasis valve as the bypass tube passes through the hemostasis valve. Fig. 10 illustrates a cross-section of the hemostasis valve assembly 200 assembled to the hemostasis valve knob 300 in the distal or bypassed condition. The hemostasis valve assembly 200 shown in Fig. 10 is identical to the embodiment shown in Figs. 3A-E with a single exception, which is that the interior flange 230 includes a generally conical tapered portion 230a that tapers in the proximal direction. Although the tapered section 230a is illustrated as two arms in Fig. 10 due to the view being a cross-section, the tapered section 230a may in reality be a frustocone with the diameter increasing in the distal direction, and the proximal opening of the tapered section 230a having a diameter substantially equal to the outer diameter of the bypass tube 380. Although not shown, an O-ring, gasket, or other seal may be provided on the interior surface of the tapered section 230a so that an additional seal is provided between the bypass tube 380 and the tapered section 230a. With this configuration, whether the bypass tube 380 is in the proximal or distal position, the bypass tube 380 is always forced to be coaxial with the center of the hemostasis valve 240. Thus, as the bypass tube 380 is advanced through the hemostasis valve, it is forced to remain coaxial with the hemostasis valve 240, which may reduce the likelihood of the bypass tube 380 tearing or otherwise damaging the hemostasis valve 380 as it passes through. Further, when the bypass tube 380 is in the proximal condition and not passing through the hemostasis valve 240, the fact that the bypass tube 380 remains securely coaxial with the hemostasis valve 240 helps to ensure that any other components passing through the bypass tube 380, such as dilator 700, remain coaxial with the hemostasis valve 240 as that other component passes through the hemostasis valve 240. It should be understood that the tapered section 230a may be included in other embodiments, including for example with the "push-to-bypass" configurations described herein.

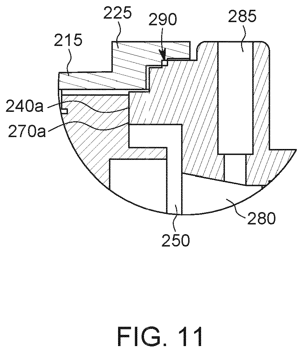

Another situation that may increase risk of air ingress through hemostasis valve 240 is if the hemostasis valve 240 is compressed too much when the hemostasis valve assembly 200 is in the assembled state. For example, referring briefly back to Fig. 3E, the point of contact between the distal end of hemostasis valve 240 and the proximal end of hub 270 may be via a proximally protruding portion of the hub 240. This proximally protruding portion of the hub 270 may help to secure the hemostasis valve 240 via compression, but if the compression is too large, the hemostasis valve 240 may not seal correctly, and/or may not return to a closed sealed position after a device (e.g. dilator 700) is withdrawn from the hemostasis valve 240. One way to help secure hemostasis valve 240 with less compression (or at least less localized compression) is by including a flat surface 270a on the proximal end of hub 270. As shown in Fig. 11, this will result in a generally flat contact between a distal flat surface 240a of the hemostasis valve 240 and the proximal flat surface 270a of the hub 270. When assembled, the hemostasis valve 240 may still be compressed slightly, but the flat contact may better distribute the forces and be less likely to result in the hemostasis valve 240 failing to close (or failing to remain closed). Other variations may be provided that help secure the hemostasis valve 240 without compressing (or overly-compressing) the hemostasis valve 240. For example, the hemostasis valve 240 may include grooves or recesses in the proximal and/or distal surfaces, while the proximal hub 270 and the distal face of the interior flange 230 may include complementary protrusions that are received within the grooves or recesses (or vice versa). It should be understood that the configuration shown in connection with Fig. 11 may be used with any embodiment herein, whether a "twist-to-bypass" or "push-to-bypass" type of configuration.

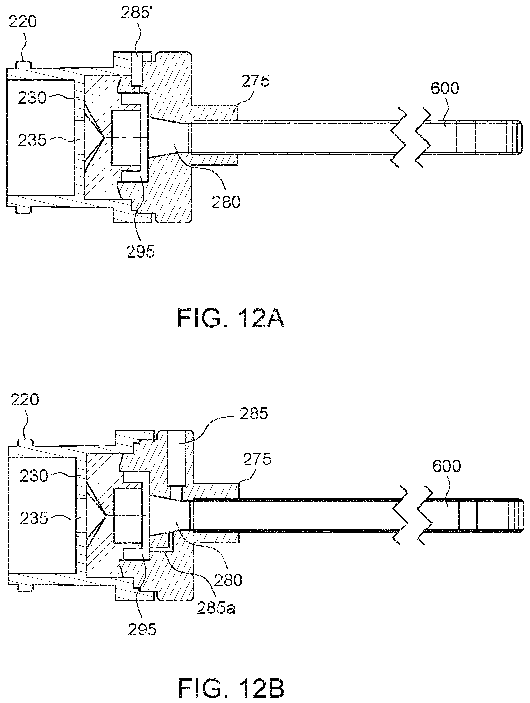

In addition to avoiding air ingress during use, it is generally desirable to be able to purge all or nearly all air from the system prior to use. Small voids within the system outside the main path of the flush port(s) may create difficulty in fully purging all of the air prior to use. For example as described above in connection with Fig. 3D, a generally cylindrical recess 295 may be positioned between the hemostasis valve 240 and the hub 270. In embodiments shown above, this cylindrical recess 295 is positioned proximal to the inlet of the flush port 285, which may create difficulties in purging the air within the cylindrical recess 295. As shown in Fig. 12A, the flush port may be moved, or a secondary flush port may be provided, that leads directly to the cylindrical recess 295. For example, as shown in Fig. 12A, the flush port 285' is formed partially by the rim 225 of the main body 220, and partially by the hub 270, with the flush port 285' directly leading to the cylindrical recess 295. In other embodiments, the flush port may be formed only in the hub 270 and still lead directly to the cylindrical recess 295, including for example by having an angled flush port or by including an interior fluid pathway leading to the cylindrical recess 295. In another embodiment, shown in Fig. 12B, one or more additional channels 285a may be introduced into the hub 270 that lead to the cylindrical recess 295, for example from central aperture 280. With this embodiment, as fluid is pushed through flush port 285, it may be routed directly into the cylindrical recess 295 to help purge any air remaining therein. It should be understood that the configurations shown in connection with Figs. 12A-B may be used with any embodiment herein, whether a "twist-to-bypass" or "push-to-bypass" type of configuration.

Although stated throughout the above description, is it repeated here that the various components and embodiments may be combined in ways other than explicitly shown without departing from the scope of the disclosure. For example, either example of wiper seal (and even other specific configurations of wiper seals not explicitly described in detail herein) may be used with any of the "twist-to-bypass" or "push-to-bypass" embodiments described herein, and in some embodiments both wiper seals may be used with any of the "twist-to-bypass" or "push-to-bypass" embodiments described herein.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised.

## Claims

1. A delivery device (10) comprising:
a handle (100);
a catheter sheath (600) extending distally from the handle (100);
a hemostasis valve assembly (200) coupled to a proximal end of the handle, a hemostasis valve (240, 2240) positioned within the hemostasis valve assembly (200), the hemostasis valve (240, 2240) being located proximal to the catheter sheath (600) and distal to a proximal end of the hemostasis valve assembly (200);
a hemostasis bypass assembly coupled to the hemostasis valve assembly (200), the hemostasis bypass assembly including a bypass tube (380, 2380) coupled to an actuator (300, 2300), the actuator (300, 2300) being a rotatable knob and being configured to be transitioned between a first condition in which a distal end of the bypass tube (380, 2380) is positioned proximal to the hemostasis valve (240, 2240) and the hemostasis valve (240, 2240) is closed, and a second condition in which the distal end of the bypass tube (380, 2380) traverses the hemostasis valve (240, 2240) and the hemostasis valve (240, 2240) is opened, and
a wiper seal (3000, 4000) positioned within the hemostasis valve assembly (200) proximal to the hemostasis valve (240, 2240),
wherein the hemostasis valve assembly (200) includes a main body (210) extending proximal of the hemostasis valve (240) and a hub (270) extending distally of the hemostasis valve (240),
wherein the main body (210) includes one or more pins (220) extending radially outwardly from the main body (210), and the rotatable knob (300) includes one or more enclosed slots (1324, 1324'), the one or more pins (220) received within a corresponding one or more of the enclosed slots (1324, 1324'), such that rotation of the rotatable knob (300) translates bypass tube (380) toward or away from the hemostasis valve (240),
wherein each of the one or more enclosed slots (1324, 1324') includes a central portion (1324a, 1324a') extending at an oblique angle relative to a central longitudinal axis of the rotatable knob (300),
wherein each of the one or more enclosed slots (1324, 1324') includes a proximal end portion (1324b, 1324b') and a distal end portion (1324c), the central portion (1324a, 1324a') extending between the proximal end portion (1324b, 1324b') and the distal end portion (1324c),
**characterised in that** the proximal end portion (1324b') of each of the one or more enclosed slots (1324') extends in a circumferential direction around the rotatable knob (300) with a pocket extending distally in an axial direction, the pocket configured to secure a corresponding one of the one or more pins (220) therein.

2. The delivery device (10) of claim 1, wherein the hemostasis valve assembly (200) includes an interior flange (230) defining a central aperture (235) that is coaxial with the bypass tube (380).

3. The delivery device (10) of claim 2, wherein the wiper seal (4000) is coupled to a distal face of the interior flange (230), the wiper seal (4000) defining a seal opening (4300) coaxial with the central aperture (235) of the interior flange (230).

4. The delivery device of claim 3, wherein the wiper seal (4000) is a flat wiper seal (4000).

5. The delivery device (10) of claim 2, wherein the wiper seal (3000) is coupled to a proximal face of the interior flange (230), the wiper seal (3000) defining a seal opening (3300) coaxial with the central aperture (235) of the interior flange (230).

6. The delivery device (10) of claim 5, wherein the wiper seal (3000) includes a rim (3400) and a proximally contoured main body (3200) defining the seal opening (3300).

7. The delivery device of any of claim 1, wherein each of the one or more enclosed slots (1324, 1324') is formed only on an interior of the rotatable knob (300).

8. The delivery device of any of claim 1, wherein each of the one or more enclosed slots (1324, 1324') is formed through an entire thickness of a wall of the rotatable knob (300).

## Patentansprüche

1. Eine Zuführvorrichtung (10) mit:
einem Griff (100);
einer Katheterhülse (600), die sich distal vom Griff (100) erstreckt;
einer Hämostase-Ventil-Baugruppe (200), die mit einem proximalen Ende des Griffs verbunden ist, einem Hämostaseventil (240, 2240), das innerhalb der Hämostase-Ventil-Baugruppe (200) positioniert ist, wobei sich das Hämostaseventil (240, 2240) proximal zur Katheterhülse (600) und distal zu einem proximalen Ende der Hämostase-Ventil-Baugruppe (200) befindet;
eine Hämostase-Bypass-Baugruppe, die mit der Hämostase-Ventil-Baugruppe (200) verbunden ist, wobei die Hämostase-Bypass-Baugruppe einen Bypassschlauch (380, 2380) aufweist, das mit einem Aktuator (300, 2300) verbunden ist, wobei der Aktuator (300, 2300) ein drehbarer Knopf ist und so konfiguriert ist, dass er zwischen einem ersten Zustand, in dem ein distales Ende des Bypassschlauchs (380, 2380) proximal zum Hämostaseventil (240, 2240) positioniert ist und das Hämostaseventil (240, 2240) geschlossen ist, und einem zweiten Zustand, in dem das distale Ende des Bypassschlauchs (380, 2380) das Hämostaseventil (240, 2240) durchquert und das Hämostaseventil (240, 2240) geöffnet ist, und eine Wischdichtung (3000, 4000), die innerhalb der Hämostase-Ventil-Baugruppe (200) proximal zum Hämostaseventil (240, 2240) positioniert ist,
wobei die Hämostase-Ventil-Baugruppe (200) einen Hauptkörper (210) aufweist, der sich proximal zum Hämostaseventil (240) erstreckt, und eine Nabe (270), die sich distal zum Hämostaseventil (240) erstreckt,
wobei der Hauptkörper (210) einen oder mehrere Stifte (220) aufweist, die sich radial nach außen vom Hauptkörper (210) erstrecken, und der drehbare Knopf (300) einen oder mehrere umschlossene Schlitze (1324, 1324') aufweist, wobei der eine oder die mehreren Stifte (220) in einem entsprechenden einen oder mehreren der umschlossenen Schlitze (1324, 1324') aufgenommen sind, so dass eine Drehung des drehbaren Knopfs (300) den Bypassschlauch (380) in Richtung auf das Hämostaseventil (240) zu oder von diesem weg verschiebt,
wobei jeder der einen oder mehreren umschlossenen Schlitze (1324, 1324') einen mittleren Abschnitt (1324a, 1324a') aufweist, der sich in einem schrägen Winkel relativ zu einer mittleren Längsachse des drehbaren Knopfs (300) erstreckt,
wobei jeder der einen oder mehreren umschlossenen Schlitze (1324, 1324') einen proximalen Endabschnitt (1324b, 1324b') und einen distalen Endabschnitt (1324c) aufweist, wobei sich der mittlere Abschnitt (1324a, 1324a') zwischen dem proximalen Endabschnitt (1324b, 1324b') und dem distalen Endabschnitt (1324c) erstreckt,
**dadurch gekennzeichnet, dass** sich der proximale Endabschnitt (1324b') jedes der einen oder mehreren umschlossenen Schlitze (1324') in Umfangsrichtung um den drehbaren Knopf (300) erstreckt, wobei sich eine Tasche in axialer Richtung distal erstreckt, die so konfiguriert ist, dass sie einen entsprechenden der einen oder mehreren Stifte (220) darin sichert.

2. Die Zuführvorrichtung (10) nach Anspruch 1, wobei die Hämostase-Ventil-Baugruppe (200) einen Innenflansch (230) aufweist, der eine zentrale Öffnung (235) definiert, die koaxial zum Bypassschlauch (380) ist.

3. Die Zuführvorrichtung (10) nach Anspruch 2, wobei die Abstreifdichtung (4000) mit einer distalen Fläche des Innenflansches (230) verbunden ist, wobei die Abstreifdichtung (4000) eine Dichtungsöffnung (4300) definiert, die koaxial zur zentralen Öffnung (235) des Innenflansches (230) ist.

4. Die Zuführvorrichtung nach Anspruch 3, wobei die Abstreifdichtung (4000) eine flache Abstreifdichtung (4000) ist.

5. Die Zuführvorrichtung (10) nach Anspruch 2, wobei die Abstreifdichtung (3000) mit einer proximalen Fläche des Innenflansches (230) verbunden ist, wobei die Abstreifdichtung (3000) eine Dichtungsöffnung (3300) definiert, die koaxial zur zentralen Öffnung (235) des Innenflansches (230) ist.

6. Die Zuführvorrichtung (10) nach Anspruch 5, wobei die Abstreifdichtung (3000) einen Rand (3400) und einen proximal konturierten Hauptkörper (3200) aufweist, der die Dichtungsöffnung (3300) definiert.

7. Die Zuführvorrichtung nach einem der Ansprüche 1, wobei jeder der einen oder mehreren umschlossenen Schlitze (1324, 1324') nur an einer Innenseite des drehbaren Knopfs (300) ausgebildet ist.

8. Die Zuführvorrichtung nach einem der Ansprüche 1, wobei jeder der einen oder mehreren umschlossenen Schlitze (1324, 1324') durch die gesamte Dicke einer Wand des drehbaren Knopfs (300) hindurch ausgebildet ist.

## Revendications

1. Une dispositif d'administration (10) comprenant :
une poignée (100) ;
une gaine de cathéter (600) s'étendant de manière distale à partir de la poignée (100) ;
un ensemble de valve hémostatique (200) couplé à une extrémité proximale de la poignée, une valve hémostatique (240, 2240) positionnée à l'intérieur de l'ensemble de valve hémostatique (200), la valve hémostatique (240, 2240) étant située proximale de la gaine de cathéter (600) et distale d'une extrémité proximale de l'ensemble de valve hémostatique (200);
un ensemble de dérivation hémostatique couplé à l'ensemble de valve hémostatique (200), l'ensemble de dérivation hémostatique comprenant un tube de dérivation (380, 2380) couplé à un actionneur (300, 2300), l'actionneur (300, 2300) étant un bouton rotatif et étant configuré pour passer d'un premier condition dans lequel une extrémité distale du tube de dérivation (380, 2380) est positionnée à proximité de la valve hémostatique (240, 2240) et la valve hémostatique (240, 2240) est fermée, et une deuxième condition dans laquelle l'extrémité distale du tube de dérivation (380, 2380) traverse la valve hémostatique (240, 2240) et la valve hémostatique (240, 2240) est ouverte, et un joint racleur (3000, 4000) positionné à l'intérieur de l'ensemble de valve hémostatique (200) proximale de la valve hémostatique (240, 2240),
dans lequel l'ensemble de valve hémostatique (200) comprend un corps principal (210) s'étendant proximale de la valve hémostatique (240) et un moyeu (270) s'étendant distalement de la valve hémostatique (240),
dans lequel le corps principal (210) comprend une ou plusieurs broches (220) s'étendant radialement vers l'extérieur à partir du corps principal (210), et le bouton rotatif (300) comprend une ou plusieurs fentes fermées (1324, 1324'), la ou les broches (220) étant reçues dans une ou plusieurs fentes fermées correspondantes (1324, 1324'), de telle sorte que la rotation du bouton rotatif (300) déplace le tube de dérivation (380) vers ou loin de la valve hémostatique (240),
dans lequel chacune des une ou plusieurs fentes fermées (1324, 1324') comprend une partie centrale (1324a, 1324a') s'étendant selon un angle oblique par rapport à un axe longitudinal central du bouton rotatif (300),
dans lequel chacune des une ou plusieurs fentes fermées (1324, 1324') comprend une partie d'extrémité proximale (1324b, 1324b') et une partie d'extrémité distale (1324c), la partie centrale (1324a, 1324a') s'étendant entre la partie d'extrémité proximale (1324b, 1324b') et la partie d'extrémité distale (1324c),
**caractérisé en ce que** la partie d'extrémité proximale (1324b') de chacune des une ou plusieurs fentes fermées (1324') s'étend dans une direction circonférentielle autour du bouton rotatif (300) avec une poche s'étendant distalement dans une direction axiale, la poche étant configurée pour fixer une broche correspondante parmi les une ou plusieurs broches (220) à l'intérieur.

2. Le dispositif d'administration (10) selon la revendication 1, dans lequel l'ensemble de valve hémostatique (200) comprend une bride intérieure (230) définissant une ouverture centrale (235) qui est coaxiale avec le tube de dérivation (380).

3. Le dispositif d'administration (10) selon la revendication 2, dans lequel le joint racleur (4000) est couplé à une face distale de la bride intérieure (230), le joint racleur (4000) définissant une ouverture de joint (4300) coaxiale à l'ouverture centrale (235) de la bride intérieure (230).

4. Le dispositif d'administration selon la revendication 3, dans lequel le joint racleur (4000) est un joint racleur plat (4000).

5. Le dispositif d'administration (10) selon la revendication 2, dans lequel le joint racleur (3000) est couplé à une face proximale de la bride intérieure (230), le joint racleur (3000) définissant une ouverture de joint (3300) coaxiale à l'ouverture centrale (235) de la bride intérieure (230).

6. Le dispositif d'administration (10) selon la revendication 5, dans lequel le joint racleur (3000) comprend un rebord (3400) et un corps principal profilé de manière proximale (3200) définissant l'ouverture de joint (3300).

7. Le dispositif d'administration selon l'une quelconque des revendications 1, dans lequel chacune des une ou plusieurs fentes fermées (1324, 1324') est formée uniquement à l'intérieur du bouton rotatif (300).

8. Le dispositif d'administration selon l'une quelconque des revendications 1, dans lequel chacune des une ou plusieurs fentes fermées (1324, 1324') est formée à travers toute l'épaisseur d'une paroi du bouton rotatif (300).
